# DEMANDE DE BREVET EUROPEEN

(11) **EP 1 844 756 A1**
(43) Date de publication de la demande: **17.10.2007**
(21) Numéro de dépôt: 07106025.5
(22) Date de dépôt: 12.04.2007
(51) Int. Cl.: A61K 8/40, A61K 8/87, A61K 8/81, A61Q 5/00

(54) **Produit cosmétique comprenant au moins un monomère cyanoacrylate et au moins un polyuréthane non-ionique.**

(30) Priorité: 13.04.2006 FR 0603286
(71) Demandeur: L'Oréal, 75008 Paris (FR)
(72) Inventeur: LIVOREIL, Aude, 75006, PARIS (FR); BRUN, Gaëlle, 75015, PARIS (FR)
(74) Mandataire: Dossmann, Gérard

(57) **Abrégé**

La présente invention concerne un produit cosmétique pour le traitement des fibres kératiniques, en particulier des fibres kératiniques humaines telles que les cheveux, comprenant au moins deux composés, caractérisé en ce qu'il comprend :
- comme premier composé, un monomère cyanoacrylate polymérisable et
- comme deuxième composé, un polyuréthane non ionique,
lesdits composés étant présents dans une même composition ou sous forme séparée, et étant destinés à être appliqués au moment de l'emploi, soit conjointement, soit séparément, de façon simultanée ou séquentielle dans le temps.

La présente invention porte notamment sur ledit produit se présentant sous la forme d'une composition, sur des procédés de traitement et de coloration mettant en oeuvre ladite composition et sur l'utilisation de celle-ci.

## Description

La présente invention concerne un produit cosmétique pour le traitement des fibres kératiniques, en particulier des fibres kératiniques humaines telles que les cheveux, comprenant un monomère cyanoacrylate et un polyuréthane non-ionique.

Elle porte, en particulier, sur ledit produit cosmétique, se présentant sous la forme d'une composition, sur une utilisation de cette composition pour le traitement des cheveux, ainsi qu'un procédé de traitement mettant en oeuvre ladite composition.

Les cheveux sont généralement abîmés et fragilisés par l'action des agents atmosphériques extérieurs tels que la lumière et les intempéries, et par des traitements mécaniques ou chimiques tels que le brossage, le peignage, les décolorations, les permanentes et/ou les teintures. Il en résulte que les cheveux sont souvent difficiles à discipliner, en particulier ils sont difficiles à démêler ou à coiffer, et les chevelures, même abondantes, conservent difficilement une coiffure de bon aspect en raison du fait que les cheveux manquent de vigueur, de volume et de nervosité.

Ainsi, pour remédier à cela, il est maintenant usuel d'utiliser des produits de coiffage qui permettent de conditionner les cheveux en leur apportant notamment du corps, de la masse ou du volume.

Ces produits de coiffage sont généralement des compositions cosmétiques capillaires comprenant un ou plusieurs polymères qui présentent une forte affinité pour les cheveux et qui ont le plus souvent pour fonction de former un film à leur surface en vue de modifier leurs propriétés superficielles, notamment pour les conditionner.

Un inconvénient lié à l'utilisation de ces compositions capillaires réside dans le fait que les effets cosmétiques conférés par de telles compositions ont tendance à disparaître, notamment dès le premier shampooing.

Afin de remédier à cet inconvénient, il est théoriquement possible d'accroître la rémanence du dépôt de polymères en effectuant directement une polymérisation radicalaire de certains monomères au niveau des cheveux.

Toutefois, les traitements ainsi obtenus sont inacceptables au point de vue cosmétique. En effet, on constate généralement une forte dégradation de la fibre liée probablement à la présence des amorceurs de polymérisation et les cheveux ainsi traités sont difficilement démêlables.

Il est connu du document FR-A-2 833 489 d'utiliser des monomères cyanoacrylate polymérisant par voie anionique directement à la surface du cheveu en présence d'un agent nucléophile tel que des ions hydroxyde (OH⁻) contenus dans l'eau à pH neutre. Ainsi, une fois déposés sur la chevelure, ces monomères forment un polymère conduisant à un conditionnement rémanent aux shampooings.

Cependant, il a été observé que les propriétés cosmétiques obtenues à partir de ce procédé n'étaient pas satisfaisantes.

Il existe donc un réel besoin de trouver des compositions cosmétiques, notamment pour le conditionnement des cheveux, qui soient rémanentes aux shampooings tout en conservant de bonnes propriétés cosmétiques, c'est-à-dire d'apporter du corps, de la masse, du volume aux cheveux et ceci de manière durable.

De manière surprenante et avantageuse, la Demanderesse vient de découvrir qu'en associant des polyuréthanes non-ioniques à un monomère cyanoacrylate polymérisable dans des proportions particulières, il était possible de résoudre ces problèmes.

La demanderesse a plus particulièrement constaté qu'en appliquant sur la chevelure une telle composition, il se formait in situ un revêtement ou un gainage rémanent. Sans que cette explication soit limitative, il semble que ce soit les ions hydroxyde (OH⁻) contenus dans l'eau à pH neutre absorbée par les cheveux qui enclenchent le processus de polymérisation anionique à l'interface composition de traitement-cheveux. Le polyuréthane présent dans la composition se trouve ainsi imbriqué dans la structure polymérique obtenue, ce qui permet d'améliorer les propriétés cosmétiques de la chevelure.

Ainsi, une telle composition permet d'améliorer les propriétés cosmétiques de la chevelure, et ceci de manière durable.

La présente invention a donc pour objet un produit cosmétique pour le traitement des fibres kératiniques, en particulier des fibres kératiniques humaines telles que les cheveux, comprenant au moins deux composés, caractérisé en ce qu'il comprend :
- comme premier composé, un monomère cyanoacrylate polymérisable, et
- comme deuxième composé, un polyuréthane non ionique,
lesdits composés étant présents dans une même composition ou sous forme séparée, et étant destinés à être appliqués, au moment de l'emploi, soit conjointement, soit séparément, de façon simultanée ou séquentielle dans le temps.

En particulier, elle porte notamment sur un produit cosmétique pour le traitement des fibres kératiniques, en particulier des fibres kératiniques humaines telles que les cheveux, caractérisé par le fait qu'il comprend dans une même composition, au moins un monomère cyanoacrylate polymérisable et au moins un polyuréthane non-ionique.

L'invention a aussi pour objet un procédé de traitement cosmétique mettant en oeuvre la composition cosmétique selon l'invention.

Un autre objet de la présente invention consiste en une utilisation de la composition cosmétique pour le traitement des cheveux.

En particulier, l'invention concerne une utilisation de la composition cosmétique pour le conditionnement des cheveux.

Plus particulièrement encore, l'invention concerne une utilisation de la composition cosmétique pour la coloration des cheveux.

L'invention a encore pour objet des dispositifs à plusieurs compartiments ou kits.

D'autres objets, caractéristiques, aspects et avantages de l'invention apparaîtront encore plus clairement à la lecture de la description et des exemples qui suivent.

Le ou les monomères cyanoacrylate présents dans la composition de l'invention sont de préférence choisis parmi les monomères de formule (I) : 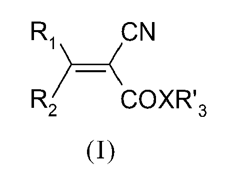 dans laquelle :
X désigne NH, S ou O,
R₁ et R₂ désignent chacun, indépendamment l'un de l'autre, un groupe peu ou non électro-attracteur (peu ou non inductif-attracteur) tel que :
   - un atome d'hydrogène,
   - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH et les atomes d'halogène,
   - un résidu polyorganosiloxane modifié ou non,
   - un groupement polyoxyalkylène.

R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en C₁-C₁₀.

Par groupement électro-attracteur ou inductif-attracteur (-I), on entend tout groupement plus électronégatif que le carbone. On pourra se reporter à l'ouvrage PR Wells Prog. Phys. Org. Chem., Vol. 6, p. 111 (1968).

Par groupement peu ou non électro-attracteur, on entend tout groupement dont l'électronégativité est inférieure ou égale à celle du carbone.

Les groupements alcényle ou alcynyle ont de préférence de 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, on peut notamment citer les groupes alkyle, alcényle ou alcynyle, linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence de 1 à 200 motifs oxyalkylénés.

Parmi les groupements mono- ou polyfluoroalkyle, on peut notamment citer des groupements tels que -(CH₂)ₙ-(CF₂)ₘ-CF₃ ou -(CH₂)ₙ-(CF₂)ₘ-CHF₂ avec n un nombre entier allant de 1 à 20 et m un nombre entier allant de 1 à 20.

Les substituants R₁ et R₂ peuvent éventuellement être substitués par un groupement ayant une activité cosmétique. Les activités cosmétiques particulièrement utilisées sont obtenues à partir de groupements à fonctions colorantes, antioxydantes, de filtres UV et conditionnantes.

A titre d'exemples de groupement à fonction colorante, on peut notamment citer les groupements azoïques, quinoniques, méthiniques, cyanométhiniques et triarylméthane.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction de filtres UV, on peut notamment citer les groupements de type benzophénone, cinnamate, benzoate, benzylidène-camphre et dibenzoylméthane.

A titre d'exemples de groupement à fonction conditionnante, on peut notamment citer les groupements cationiques et de type ester gras.

De préférence, R₁ et R₂ représentent un atome d'hydrogène.

R'₃ représente un atome d'hydrogène ou un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en C₁-C₁₀.

De préférence, R'₃ est un groupe hydrocarboné saturé comportant de 1 à 10 atomes de carbone ou un groupe alcènyle en C₂-C₁₀.

De préférence, X désigne O.

A titre de composés de formule (I), on peut citer les monomères :
a) appartenant à la famille des 2-cyanoacrylate de polyfluoroalkyle tels que :
   l'ester 2,2,3,3-tétrafluoropropylique de l'acide 2-cyano-2-propénoïque de formule : 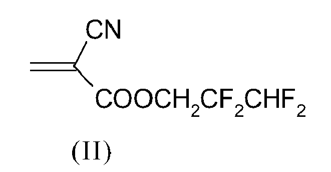
   ou encore l'ester 2,2,2-trifluoroéthylique de l'acide 2-cyano-2-propénoïque de formule : 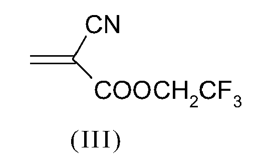
b) appartenant à la famille des 2-cyanoacrylates d'alkyle ou d'alcoxyalkyle 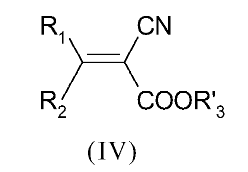 dans laquelle R'₃ représente un radical alkyle en C₁-C₁₀ ou alcoxy(C₁-C₄) alkyle(C₁-C₁₀).

On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate d'allyle, le 2-cyanoacrylate de 2-méthoxypropyle, le 2-éthoxyéthyle, le 2-cyanoacrylate de 2-méthoxyéthyle, le 2-cyanoacrylate de 2-méthylheptyle, le 2-cyanoacrylate de 2-propoxyéthyle, le 2-cyanoacrylate de n-octyle et le cyanoacrylate d'iso-amyle.

Dans le cadre de l'invention, on préfère utiliser les monomères b). Selon un mode de réalisation préféré, le ou les monomères cyanoacrylates sont choisis parmi les cyanoacrylates d'alkyle en C₆-C₁₀.

Les monomères particulièrement préférés sont les cyanoacrylates d'octyle de formule (V) et leurs mélanges : 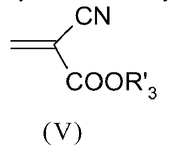
- dans laquelle : R'₃ =: -(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Dans le cadre de la présente invention, les monomères cyanoacrylate de formule (I) sont des monomères capables de polymériser par voie anionique en présence d'un agent nucléophile. Par polymérisation anionique, on entend le mécanisme défini dans l'ouvrage "Advanced Organic Chemistry", Third Edition de Jerry March, pages 151 à 161.

Les monomères cyanoacrylate de formule (I) selon la présente invention peuvent être synthétisés selon les méthodes connues décrites dans la technique. En particulier, les monomères cyanoacrylates peuvent être synthétisés selon l'enseignement des documents US 3,527,224, US 3,591,767, US 3,667,472, US 3,995,641, US 4,035,334 et US 4,650,826.

Le monomère cyanoacrylate dans la composition cosmétique conforme à l'invention est introduit de préférence en une quantité allant de 0,1 % à 99,9 % en poids, de préférence de 0,5 à 40 % en poids, par rapport au poids total de la composition.

La composition selon la présente invention comprend au moins un polyuréthane non-ionique. Ce polyuréthane peut être non-associatif ou associatif.

Par polyuréthane non associatif, on entend au sens de la présente invention, des polycondensats comprenant au moins une séquence polyuréthane et ne comprenant pas, dans leur structure, de chaîne alkyle ou alcényle, terminale ou pendante, comportant plus de 10 atomes de carbone. Ils sont décrits en particulier dans les brevets EP-A-0 751 162, EP-A-0 637 600, FR-A-2 743 297 et EP-A-0 648 485 dont la Demanderesse est titulaire, ainsi que les brevets EP-A-0 656 021 ou WO 94/03510 de la Société BASF et EP-A-0 619 111 de la Société National Starch.

Les polyuréthanes non associatifs utilisés conformément à l'invention peuvent être solubles dans le milieu aqueux cosmétiquement acceptable, ou encore former une dispersion dans ce milieu. La dispersion peut comprendre alors au moins 0,05 % de tensioactif permettant la mise en dispersion et le maintien en dispersion du polyuréthane non associatif.

Selon l'invention, on peut utiliser tout type de tensioactif dans ladite dispersion, mais de préférence un tensioactif non ionique. La taille moyenne des particules du polyuréthane non associatif dans la dispersion est de préférence comprise entre 0,1 et 1 micromètre.

A titre d'exemple, le polyuréthane non associatif peut être formé par un arrangement de blocs, cet arrangement étant obtenu notamment à partir de :
(1) au moins un composé qui contient deux ou plus de deux atomes d'hydrogène actifs par molécule ;
(2) au moins un diol ou un mélange de diols contenant des fonctions acides ou leurs sels ; et
(3) au moins un di- ou polyisocyanate.

Avantageusement, les composés (1) sont choisis dans le groupe comprenant les diols, les diamines, les polyesterols, les polyétherols, et leurs mélanges.

Les composés (1) qui sont préférés sont les polyéthylèneglycols et les polypropylèneglycols linéaires, en particulier ceux qui sont obtenus par réaction de l'oxyde d'éthylène ou de propylène avec l'eau ou du diéthylène ou du dipropylèneglycol en présence d'hydroxyde de sodium en tant que catalyseur. Ces polyalkylèneglycols ont généralement une masse moléculaire comprise entre environ 600 et 20 000.

D'autres composés organiques préférés sont ceux qui ont des groupes mercapto, amino, carboxyle ou hydroxyle. Parmi ceux-ci, on cite plus particulièrement les composés polyhydroxylés tels que les polyéther-diols, les polyester-diols, les polyacétal-diols, les polyamide-diols, les polyester-polyamide-diols, les poly(alkylène éther)-diols, les polythioéther-diols et les polycarbonate-diols.

Les polyéther-diols préférés sont, par exemple, les produits de condensation d'oxyde d'éthylène, d'oxyde de propylène ou de tétrahydrofurane, leurs produits de copolymérisation ou de condensation, greffés ou blocs, tels que les mélanges de condensats d'oxyde d'éthylène et de propylène, et les produits de polymérisation d'oléfines, sous haute pression, avec les condensats d'oxyde d'alkylène. Des polyéther-diols appropriés sont par exemple préparés par condensation d'oxydes d'alkylène et d'alcools polyhydriques, tels que l'éthylèneglycol, le 1,2-propylèneglycol et le 1,4-butanediol.

Les polyester-diols, polyester-amides, polyamide-diols sont de préférence saturés et sont obtenus, par exemple, à partir de la réaction d'acides polycarboxyliques saturés ou insaturés avec des alcools polyhydriques, des diamines ou des polyamines. Pour préparer ces composés, on peut utiliser, par exemple, l'acide adipique, l'acide succinique, l'acide phtalique, l'acide téréphtalique et l'acide maléique. Des alcools polyhydriques appropriés pour préparer les polyesters incluent par exemple l'éthylèneglycol, le 1,2-propylèneglycol, le 1,4-butanediol, le néopentylglycol et l'hexanediol. On peut aussi utiliser des aminoalcools, par exemple l'éthanolamine. Des diamines appropriées pour préparer les amide-polyesters sont l'éthylène-diamine et l'hexaméthylène-diamine.

Des polyacétals appropriés peuvent être préparés, par exemple, à partir de 1,4-butanediol ou d'hexanediol et de formaldéhyde. Des polythioéthers appropriés peuvent être préparés, par exemple, par réaction de condensation entre des thioglycols seuls ou en combinaison avec d'autres glycols tels que l'éthylèneglycol, le 1,2-propylèneglycol ou avec d'autres composés polyhydroxylés. Les composés polyhydroxylés contenant déjà des groupements uréthannes, des polyols naturels, qui peuvent être davantage modifiés, par exemple, l'huile de ricin et les carbohydrates peuvent également être utilisés.

Plus préférentiellement, le composé du groupe (1) est un polyestérol, notamment un polyester-diol formé par la réaction d'au moins un (di)-polyol (la) et d'au moins un acide (1b). Le (di)-polyol (la) est en particulier choisi dans le groupe comprenant le néopentylglycol, le 1,4-butanediol, l'hexanediol, l'éthylèneglycol, le diéthylèneglycol, le propylèneglycol, le butylèneglycol, le néopentylglycol et (di)-polyéthylèneglycol. L'acide (1b) est en particulier choisi dans le groupe comprenant l'acide phtalique, l'acide isophtalique, l'acide adipique et l'acide (poly)-lactique.

En tant que composé (2), on peut notamment utiliser un acide hydroxycarboxylique tel que l'acide diméthylol-propanoïque (DMPA) ou un acide 2,2-hydroxyméthyl-carboxylique. En général, le composé (2) est utile en tant que bloc de couplage. En tant que composés (2), on préfère ceux comprenant au moins un poly(acide carboxylique α,α-dihydroxylé).

Les composés (2) particulièrement préférés conformément à l'invention sont ceux choisis dans le groupe comprenant l'acide 2,2-di-(hydroxyméthyl)acétique, l'acide 2,2-dihydroxyméthylpropionique, l'acide 2,2-dihydroxyméthylbutyrique, l'acide 2,2-dihydroxyméthylpentanoïque.

Le di- ou polyisocyanate (3) peut être choisi en particulier dans le groupe comprenant l'hexaméthylène-diisocyanate, l'isophorone-diisocyanate (IDPI), le toluylène-diisocyanate, le diphénylméthane-4,4'-diisocyanate (DPMD) et le dicyclohexylméthane-4,4'-diisocyanate (DCMD), le méthylène-di-p-phényl-diisocyanate, le méthylène-bis(4-cyclohexylisocyanate), les toluène-diisocyanates, le 1,5-naphtalène-diisocyanate, le 4,4'-diphénylméthane-diisocyanate, le 2,2'-diméthyl-4,4'-diphénylméthane-diisocyanate, le 1,3-phénylène-diisocyanate, le 1,4-phénylène diisocyanate, des mélanges de 2,4- et de 2,6-toluène-diisocyanates, le 2,2'-dichloro-4,4'-diisocyanato-diphénylméthane, le 2,4-dibromo-1,5-diisocyanato naphtalène, le butane-1,4-diisocyanate, l'hexane-1,6-diisocyanate, et le cyclohexane-1,4-diisocyanate.

Le polyuréthane non associatif peut être formé à l'aide d'un composé supplémentaire (4) servant en général à allonger sa chaîne. Ces composés (4) peuvent être choisis dans le groupe comprenant notamment les glycols saturés ou insaturés tels que l'éthylèneglycol, le diéthylèneglycol, le néopentylglycol, le triéthylène glycol ; les aminoalcools tels que l'éthanolamine, la propanolamine, la butanolamine ; les amines primaires hétérocycliques, aromatiques, cycloaliphatiques, et aliphatiques ; les diamines ; les acides carboxyliques tels que les acides carboxyliques aliphatiques, aromatiques et hétérocycliques comme les acides oxalique, succinique, glutarique, adipique, sébacique et téréphtalique ; et les acides aminocarboxyliques. Les composés (4) préférés sont les diols aliphatiques.

Les polyuréthanes non associatifs utilisés selon l'invention peuvent également être encore formés à partir de composés supplémentaires (5) ayant un squelette siliconé tels que les polysiloxanes, les polyalkylsiloxanes ou les polyarylsiloxanes, notamment les polyéthylsiloxanes, les polyméthylsiloxanes et les polyphénylsiloxanes, comportant éventuellement des chaînes hydrocarbonées greffées sur les atomes de silicium.

Les polyuréthanes non associatifs utilisés avantageusement comprennent un motif répétitif de base répondant à la formule générale (VI) :

- O - B - O - CO - NH - R - NH - CO - (VI)

dans laquelle :
- B est un groupe hydrocarboné divalent en C₁ à C₃₀, ce groupe étant substitué ou non par un groupement comportant une ou plusieurs fonctions acides carboxyliques et/ou une ou plusieurs fonctions acides sulfoniques, lesdites fonctions acides carboxyliques et/ou sulfoniques étant sous forme libre ou bien neutralisées partiellement ou totalement par une base minérale ou organique, et
- R est un groupe divalent choisi parmi les groupes hydrocarbonés aliphatiques en C₁ à C₂₀, cycloaliphatiques en C₃ à C₂₀ et aromatiques en C₆ à C₂₀, comme par exemple les groupes alkylène en C₁ à C₂₀, arylène en C₆ à C₂₀, cycloalkylène en C₃ à C₂₀, ou leurs combinaisons, ces groupes étant substitués ou non.

Le groupe R est avantageusement choisi parmi les groupes répondant aux formules suivantes :

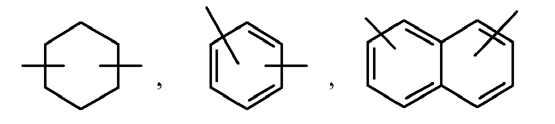

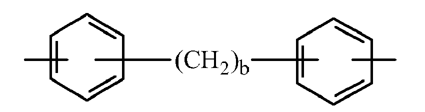

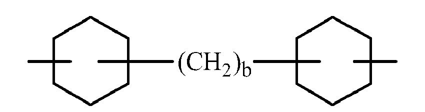

-(CH₂)_{c}-

dans lesquelles b est un nombre entier compris entre 0 et 3, et c un nombre entier compris entre 1 et 20, de préférence compris entre 2 et 12.

En particulier, le groupe R est choisi parmi les groupes hexaméthylène, 4,4'-biphénylèneméthane, 2,4- et/ou 2,6-tolylène, 1,5-naphtylène, p-phénylène, méthylène- 4,4-bis-cyclohexyle et le groupe divalent dérivé de l'isophorone.

Le polyuréthane non associatif utilisé dans la présente invention peut avantageusement comprendre en outre au moins une séquence polysiloxane dont le motif répétitif de base répond par exemple à la formule générale (VII) :

- O - P - O - CO - NH - R - NH - CO - (VII)

dans laquelle :
- P est un segment polysiloxanique, et
- R est un groupe divalent choisi parmi les groupes hydrocarbonés aliphatiques en C₁ à C₂₀, cycloaliphatiques en C₃ à C₂₀ et aromatiques en C₆ à C₂₀, comme par exemple les groupes alkylène en C₁ à C₂₀, arylène en C₆ à C₂₀, cycloalkylène en C₃ à C₂₀, ou leurs combinaisons, ces groupes étant substitués ou non.

Avantageusement, le segment polysiloxanique P répond à la formule générale (VIII) ci-après : 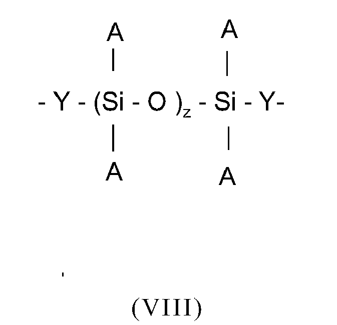 dans laquelle:
- les groupes A, qui peuvent être identiques ou différents, sont choisis parmi, d'une part, les groupes hydrocarbonés monovalents en C₁-C₂₀ exempts ou substantiellement exempts d'insaturation éthylénique et, d'autre part, les groupes aromatiques,
- Y représente un groupe hydrocarboné divalent, et
- z représente un nombre entier, choisi de telle sorte que la masse moléculaire moyenne en poids du segment polysiloxane soit comprise entre 300 et 10 000.

De préférence, le groupe divalent Y est choisi parmi les groupes alkylène de formule -(CH₂)ₐ-, dans laquelle a représente un nombre entier pouvant être compris entre 1 et 10.

Les groupes A peuvent être choisis parmi les groupes alkyle en C₁-C₈, en particulier les groupes méthyle, éthyle, propyle, isopropyle, butyle, pentyle, hexyle, octyle ; les groupes cycloalkyle en C₃-C₈, en particulier le groupe cyclohexyle ; les groupes aryle en C₆-C₁₀, notamment phényle ; les groupes arylalkyle en C₇-C₁₀, notamment benzyle et phényléthyle, ainsi que les groupes tolyle et xylyle.

A titre d'exemples de polyuréthane non associatif, on peut notamment citer le copolymère acide diméthylolpropionique/isophorone-diisocyanate/néopentylglycol/polyesterdiols (connu aussi sous le nom de polyuréthane-1, appellation INCI) vendu sous la marque Luviset^{®} PUR par la société BASF, et le copolymère acide diméthylolpropionique/isophorone-diisocyanate/néopentylglycol/polyesterdiols/diamine siliconée (connu aussi sous le nom de polyuréthane-6, appellation INCI) vendu sous la marque Luviset^{®} Si PUR A par la société BASF.

Ces polyuréthanes contenus dans la composition selon l'invention sont mis en oeuvre sous forme non neutralisée, donc non-ionique.

Par polyuréthane associatif, on entend un polyuréthane possédant au moins une chaîne alkyle terminale ou pendante comportant au moins 10 atomes de carbone. Ce type de polymère est susceptible d'interagir avec lui-même ou avec des composés particuliers tels que des tensioactifs pour conduire à un épaississement du milieu.

Les polyuréthanes associatifs utilisés dans l'invention sont non-ioniques.

A titre d'exemple de polyuréthane associatif non-ionique, on peut notamment citer un copolymère acrylique soluble ou gonflable dans l'eau. Il est caractérisé par le fait qu'il comprend :
a) environ 40 à 99,5 % en poids, de préférence 30 à 65 % en poids, d'un monomère à insaturation monoéthylénique non tensioactif et
b) environ 0,5 à 60 % en poids, de préférence 10 à 50 % en poids, d'un monomère uréthane non-ionique qui est le produit de réaction d'un tensioactif non-ionique monohydrique avec un monoisocyanate à insaturation monoéthylénique.

Le copolymère doit contenir une proportion importante indiquée ci-dessus d'un monomère a) à insaturation monoéthylénique qui n'a pas de propriété tensioactive. Les monomères préférés sont ceux qui donnent des polymères insolubles dans l'eau lorsqu'ils sont homopolymérisés et sont illustrés par les acrylates et méthacrylates d'alkyle en C₁-C₄ comme l'acrylate de méthyle, l'acrylate d'éthyle, l'acrylate de butyle ou les méthacrylates correspondants. Les monomères plus particulièrement préférés sont les (méth)acrylates de méthyle et d'éthyle. D'autres monomères pouvant être utilisés sont le styrène, le vinyltoluène, l'acétate de vinyle, l'acrylonitrile et le chlorure de vinylidène. Les monomères non réactifs sont préférés, ces monomères étant ceux dans lesquels le groupe éthylénique unique est le seul groupe réactif dans les conditions de la polymérisation. Cependant, des monomères qui contiennent des groupes réactifs sous l'action de la chaleur peuvent être utilisés dans certaines situations, comme l'acrylate d'hydroxyéthyle.

Les tensioactifs non-ioniques monohydriques utilisés pour obtenir le monomère uréthane non-ionique b) sont bien connus et sont généralement des composés hydrophobes alcoxylés contenant un oxyde d'alkylène formant la partie hydrophile de la molécule. Les composés hydrophobes sont généralement constitués par un alcool aliphatique ou un alkylphénol dans lesquels une chaîne carbonée contenant au moins six atomes de carbone constitue la partie hydrophobe du tensioactif.

Les tensioactifs non-ioniques monohydriques préférés ont pour formule : 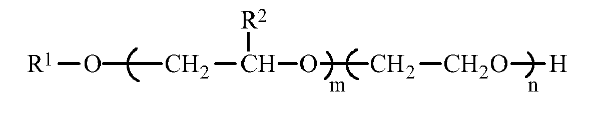 dans laquelle R¹ est un groupe alkyle en C₆-C₃₀ ou aralkyle en C₈-C₃₀, R² est un groupe alkyle en C₁-C₄, n est un nombre moyen allant d'environ 5 à 150 et m est un nombre moyen allant d'environ 0 à 50, à la condition que n soit au moins aussi grand que m et que n + m = 5 à 150.

A titre de groupes alkyle en C₆-C₃₀ préférés, on peut citer les radicaux dodécyle et alkyle en C₁₈-C₂₆. A titre de groupes aralkyle, on peut citer plus particulièrement les groupes alkyl (C₈-C₁₃) phényle. Le groupe R² préféré est le groupe méthyle.

Le monoisocyanate à insaturation monoéthylénique utilisé pour former le monomère uréthane non-ionique b) peut être choisi parmi des composés très variés. On peut utiliser un composé contenant toute insaturation copolymérisable telle qu'une insaturation acrylique ou méthacrylique. On peut aussi utiliser une insaturation allylique conférée par l'alcool allylique. Les monoisocyanates monoéthyléniques préférés sont l'α,α-diméthyl-m-isopropényl-benzylisocyanate et le méthylstyrène-isopropylisocyanate.

Le copolymère acrylique défini ci-dessus est obtenu par copolymérisation en émulsion aqueuse des composants a) et b) qui est usuelle et décrite dans la demande de brevet EP-A-0 173 109.

Les polyuréthanes associatifs non ioniques utilisés dans la présente invention sont notamment des polyuréthanes-polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, les polyuréthane-polyéthers comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyuréthane-polyéthers peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyuréthane-polyéthers non-ioniques à chaîne grasse peuvent être des copolymères triblocs dont la séquence hydrophile est une chaîne polyoxyéthoxylée comportant de 50 à 1000 groupements éthoxylés. Les polyuréthane-polyéthers non-ioniques comportent une liaison uréthanne entre les séquences hydrophiles, d'où l'origine du nom.

Par extension figurent aussi parmi les polyuréthane-polyéthers non-ioniques à chaîne grasse, ceux dont les séquences hydrophiles sont liées aux séquences lipophiles par d'autres liaisons chimiques.

A titre d'exemples de polyuréthane-polyéthers non-ioniques à chaîne grasse utilisables dans l'invention, on peut mentionner aussi le Rhéolate 205 à fonction urée vendu par la société RHEOX ou encore les Rhéolates 208, 204 ou 212,ainsi que l'Acrysol^{®} RM 184.

On peut également citer le produit ELFACOS T210 à chaîne alkyle en C₁₂-C₁₄ et le produit ELFACOS^{®} T212 à chaîne alkyle en C₁₈ de chez AKZO.

Le produit DW 1206B de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemples de tels polymères, on peut citer le Rhéolate^{®} 255, le Rhéolate^{®} 278 et le Rhéolate^{®} 244 vendus par la société RHEOX. On peut aussi utiliser le produit DW 1206F et le DW 1206J proposés par la société ROHM & HAAS.

Les polyuréthane-polyéthers utilisables selon l'invention sont en particulier ceux décrits dans l'article de G. Fonnum, J. Bakke et Fk. Hansen - Colloid Polym. Sci. 271, p. 380-389 (1993).

Comme exemples préférés de polyuréthane associatif non ionique, on peut citer les polyuréthane-polyéthers susceptibles d'être obtenus par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyuréthane-polyéthers sont vendus notamment par la société ROHM & HAAS sous les appellations Aculyn^{®} 46 et Aculyn^{®} 44. L'ACULYN^{®} 46 est un polycondensat de polyéthylèneglycol à 150 ou 180 moles d'oxyde d'éthylène, d'alcool stéarylique et de méthylène-bis(4-cyclohexylisocyanate) (SMDI), à 15 % en poids dans une matrice de maltodextrine (4 %) et d'eau (81 %) ; l'ACULYN^{®} 44 est un polycondensat de polyéthylèneglycol à 150 ou 180 moles d'oxyde d'éthylène, d'alcool décylique et de méthylène-bis(4-cyclohexylisocyanate) (SMDI), à 35 % en poids dans un mélange de propylèneglycol (39 %) et d'eau (26 %).

Le polyuréthane non ionique dans la composition cosmétique conforme à l'invention est introduit en une quantité allant de préférence de 0,1 % à 99,9 % en poids, de préférence de 0,1 à 40 % en poids.

Les agents nucléophiles susceptibles d'initier la polymérisation anionique sont des systèmes connus en eux-mêmes, capables de générer un carbanion au contact d'un agent nucléophile, tels que les ions hydroxydes contenus dans l'eau à pH neutre. On entend par " carbanion ", les espèces chimiques définies dans "Advanced Organic Chemistry, Third Edition ", de Jerry March, page 141.

Les agents nucléophiles peuvent être appliqués indépendamment de la composition de l'invention. Il peut aussi être ajouté à la composition de l'invention au moment de l'emploi.

L'agent nucléophile est un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻, H₂O ; Ph représentant un groupe phényle ; Ar représentant un groupe aryle et R représentant un groupe alkyle en C₁-C₁₀. De préférence, l'agent nucléophile est de l'eau.

La composition cosmétique selon l'invention peut comprendre en outre au moins un pigment.

L'utilisation d'un pigment dans la composition cosmétique selon l'invention permet d'obtenir des colorations visibles sur des cheveux foncés puisque le pigment en surface masque la couleur naturelle de la fibre.

La composition conforme à l'invention présente ainsi l'avantage de conduire à des colorations qui présentent une bonne résistance aux diverses agressions que peuvent subir les cheveux, telles que les corps gras ou les shampooings.

De plus, la composition cosmétique selon l'invention permet de conduire à des colorations visibles et très chromatiques sur une fibre kératinique foncée sans qu'il soit nécessaire d'éclaircir ou de décolorer les fibres kératiniques et, par conséquent, sans dégradation physique des fibres kératiniques.

Au sens de la présente invention, on entend par pigment toute entité organique et/ou minérale dont la solubilité dans l'eau est inférieure à 0,01 % à 20° C, de préférence inférieure à 0,0001 %, et présentant une absorption entre 350 et 700 nm, de préférence une absorption avec un maximum.

Les pigments utilisés dans la composition selon l'invention peuvent être notamment choisis parmi les pigments organiques et/ou minéraux connus de la technique, notamment ceux qui sont décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire. Ils peuvent être enrobés ou non enrobés.

Les pigments conformes à l'invention peuvent par exemple être choisis parmi les pigments blancs ou colorés, les laques, les pigments à effets spéciaux tels que les nacres ou les paillettes, et leurs mélanges.

A titre d'exemples de pigments minéraux blancs ou colorés, on peut citer le dioxyde de titane, traité ou non traité en surface, les oxydes de zirconium ou de cérium, les oxydes de fer ou de chrome, le violet de manganèse, le bleu outremer, l'hydrate de chrome et le bleu ferrique. Par exemple, les pigments minéraux suivants peuvent être utilisés : Ta₂O₅, Ti₃O₅, Ti₂O₃, TiO, ZrO₂ en mélange avec TiO₂, ZrO₂, Nb₂O₅, CeO₂, ZnS.

A titres d'exemples de pigments organiques blancs ou colorés, on peut citer les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR-A-2 679 771.

On peut utiliser des pâtes pigmentaires de pigment organique telles que les produits vendus par la société HOECHST sous le nom :
- JAUNE COSMENYL IOG : Pigment YELLOW 3 (CI 11710) ;
- JAUNE COSMENYL G : Pigment YELLOW 1 (CI 11680) ;
- ORANGE COSMENYL GR : Pigment ORANGE 43 (CI 71105) ;
- ROUGE COSMENYL R : Pigment RED 4 (CI 12085) ;
- CARMIN COSMENYL FB : Pigment RED 5 (CI 12490) ;
- VIOLET COSMENYL RL : Pigment VIOLET 23 (CI 51319) ;
- BLEU COSMENYL A2R : Pigment BLUE 15.1 (CI 74160) ;
- VERT COSMENYL GG : Pigment GREEN 7 (CI 74260) ;
- NOIR COSMENYL R : Pigment BLACK 7 (CI 77266).

Les pigments conformes à l'invention peuvent aussi être sous forme de pigments composites tels qu'ils sont décrits dans le brevet EP-A-1 184 426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Par laque, on entend les colorants adsorbés sur des particules insolubles, l'ensemble ainsi obtenu restant insoluble lors de l'utilisation. Les substrats inorganiques sur lesquels sont adsorbés les colorants sont par exemple l'alumine, la silice, le borosilicate de calcium et de sodium ou le borosilicate de calcium et d'aluminium, et l'aluminium. Parmi les colorants organiques, on peut citer le carmin de cochenille.

A titre d'exemples de laques, on peut citer les produits connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 7 (CI 15 850:1), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 10 (CI 77 002), D & C Green 3 (CI 42 053), D & C Blue 1 (CI 42 090).

Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments blancs ou colorés qui procurent une teinte uniforme opaque, semi-transparente ou transparente classique.

A titre d'exemples de pigments à effets spéciaux, on peut citer les pigments nacrés blancs tels que le mica recouvert de titane, ou d'oxychlorure de bismuth, les pigments nacrés colorés tels que le mica recouvert de titane et d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth, le mica recouvert d'oxyde de fer. A titre de pigments nacrés, on peut citer les nacres Cellini commercialisée par Engelhard (mica-TiO₂-laque), Prestige commercialisée par Eckart (mica-TiO₂), Colorona commercialisée par Merck (mica-TiO₂-Fe₂O₃), Prestige bronze commercialisée par Eckart (mica-Fe₂O₃), Sunshine Super Copper commercialisée par Sun Chemicals (mica-Fe₂O₃).

On peut également citer les pigments à effet interférentiel non fixés sur un substrat comme les cristaux liquides (Helicones HC de Wacker), les paillettes holographiques interférentielles (Geometrie Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être fabriquées selon les procédés décrits par exemple dans les documents US 6,225,198 ou US 5,990,479, dans les publications qui y sont citées, ainsi que dans les publications suivantes : Dabboussi B.O. et al "(CdSe)ZnS core-shell quantum dots : synthesis and characterisation of a size series of highly luminescent nanocristallites" Journal of physical chemistry B, vol. 101, 1997, p. 9463-9475 et Peng, Xiaogang et al, "Epitaxial Growth of highly Luminescent CdSe/CdS core/shell nanocrystals with photostability and electronic accessibility" Journal of the American Chemical Society, vol. 119, N° 30, p. 7019-7029.

La variété des pigments qui peuvent être utilisés dans la présente invention permet d'obtenir une riche palette de couleurs, ainsi que des effets optiques particuliers tels que des effets métalliques, interférentiels.

Selon un mode de réalisation particulier, les pigments sont des pigments colorés. On entend par pigment coloré des pigments autres que les pigments blancs.

La taille du pigment utile dans le cadre de la présente invention est généralement comprise entre 10 nm et 200 µm, de préférence entre 20 nm et 80 µm, et plus préférentiellement entre 30 nm et 50 µm.

De préférence, le pigment présent dans la composition cosmétique selon l'invention est un pigment nacré qui est le mica recouvert d'oxyde de fer.

Les pigments peuvent être enrobés par des composés organiques ou minéraux.

L'agent organique avec lequel sont traités les pigments peut être déposé sur les pigments par évaporation de solvant, réaction chimique entre les molécules de l'agent de surface ou création d'une liaison covalente entre l'agent de surface et les pigments ou les charges.

Le traitement en surface peut ainsi être réalisé par exemple par réaction chimique d'un agent de surface avec la surface des pigments et création d'une liaison covalente entre l'agent de surface et les pigments. Cette méthode est notamment décrite dans le brevet US 4,578,266.

De préférence, on utilisera un agent organique lié aux pigments ou aux charges de manière covalente.

L'agent pour le traitement de surface peut représenter de 0,1 à 50 % en poids du poids total des pigments ou des charges traités en surface, de préférence de 0,5 à 30 % en poids, et encore plus préférentiellement de 1 à 10 % en poids.

De préférence, les traitements en surface des pigments sont choisis parmi les traitements suivants :
- un traitement PEG-Silicone comme le traitement de surface AQ commercialisé par LCW ;
- un traitement Chitosane comme le traitement de surface CTS commercialisé par LCW ;
- un traitement Triéthoxycaprylylsilane comme le traitement de surface AS commercialisé par LCW ;
- un traitement Méthicone comme le traitement de surface SI commercialisé par LCW ;
- un traitement Diméthicone comme le traitement de surface Covasil 3.05 commercialisé par LCW ;
- un traitement Diméthicone / Triméthylsiloxysilicate comme le traitement de surface Covasil 4.05 commercialisé par LCW ;
- un traitement Lauroyl Lysine comme le traitement de surface LL commercialisé par LCW ;
- un traitement Lauroyl Lysine Diméthicone comme le traitement de surface LL / SI commercialisé par LCW ;
- un traitement Myristate de Magnésium comme le traitement de surface MM commercialisé par LCW ;
- un traitement Dimyristate d'Aluminium comme le traitement de surface MI commercialisé par Miyoshi ;
- un traitement Perfluoropolyméthylisopropyl éther comme le traitement de surface FHC commercialisé par LCW ;
- un traitement Isostéaryl Sébacate comme le traitement de surface HS commercialisé par Miyoshi ;
- un traitement Disodium Stéaroyl Glutamate comme le traitement de surface NAI commercialisé par Miyoshi ;
- un traitement Diméthicone / Disodium Stéaroyl Glutamate comme le traitement de surface SA / NAI commercialisé par Miyoshi ;
- un traitement Phosphate de Perfluoroalkyle comme le traitement de surface PF commercialisé par Daito ;
- un traitement Copolymère acrylate / Diméthicone et Phosphate de Perfluoalkyle comme le traitement de surface FSA commercialisé par Daito ;
- un traitement Polyméthylhydrogène siloxane / Phosphate de Perfluoroalkyle comme le traitement de surface FS01 commercialisé par Daito ;
- un traitement Lauryl Lysine / Aluminium Tristéarate comme le traitement de surface LL-StAI commercialisé par Daito ;
- un traitement Octyltriéthylsilane comme le traitement de surface OTS commercialisé par Daito ;
- un traitement Octyltriéthylsilane / Phosphate de Perfluoroalkyle comme le traitement de surface FOTS commercialisé par Daito ;
- un traitement Copolymère Acrylate / Diméthicone comme le traitement de surface ASC commercialisé par Daito ;
- un traitement Isopropyl Titanium Triisostéarate comme le traitement de surface ITT commercialisé par Daito ;
- un traitement Cellulose Microcrystalline et Carboxyméthyl Cellulose comme le traitement de surface AC commercialisé par Daito ;
- un traitement Cellulose comme le traitement de surface C2 commercialisé par Daito ;
- un traitement copolymère Acrylate comme le traitement de surface APD commercialisé par Daito ;
- un traitement Phosphate de Perfluoroalkyle / Isopropyl Titanium Triisostéarate comme le traitement de surface PF + ITT commercialisé par Daito.

Le ou les pigments sont chacun généralement présents dans la composition conforme à l'invention dans des quantités généralement comprises entre 0,05 et 50 % en poids, de préférence de 0,1 à 35 % en poids, du poids total de la composition.

On peut également introduire dans les compositions des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, le trifluorure de bore, l'hydroquinone et ses dérivés tels que l'hydroquinone monoéthyléther, la TBHQ, la benzoquinone et ses dérivés tels que la duroquinone, le catéchol et ses dérivés tels que le t-butyl catéchol et le méthoxycatéchol, l'anisole et ses dérivés tels que le méthoxyanisole ou l'hydroxyanisole, le pyrogallol et ses dérivés, le p-méthoxyphénol, l'hydroxybutyl toluène, les alkyl sulfates, les alkyl sulfites, les alkyl sulfones, les alkyl sulfoxydes, les alkyl sulfures, les mercaptans, le 3-sulfonène et leurs mélanges. Les groupements alkyles désignent de préférence des groupements ayant 1 à 6 atomes de carbone.

On peut aussi utiliser à titre d'inhibiteur les acides minéraux ou organiques.

Ainsi la composition cosmétique selon l'invention peut également comprendre au moins un acide minéral ou organique, ce dernier ayant un ou plusieurs groupements carboxyliques ou sulfoniques, présentant un pKa compris entre 0 et 6 tels que l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique, l'acide octanoïque, l'acide heptanoïque et l'acide hexanoïque.

De préférence, l'acide acétique est utilisé.

La concentration en inhibiteur dans la composition cosmétique de l'invention peut être comprise entre 10 ppm et 30 %, et plus préférentiellement entre 10 ppm et 15 % en poids, par rapport au poids total de la composition.

La composition de l'invention peut contenir de l'eau ou un ou plusieurs solvants organiques liquides, ou un mélange d'eau et d'un ou plusieurs solvants organiques liquides.

Par solvant organique, on entend une substance organique capable de dissoudre une autre substance sans la modifier chimiquement.

Les solvants organiques sont choisis parmi les composés liquides à la température de 25° C et sous 105 Pa (760 mm de Hg).

Dans le cadre de l'invention, le monomère cyanoacrylate et le solvant organique sont des composés distincts.

La composition de l'invention peut contenir au moins un solvant organique liquide.

Le solvant organique est par exemple choisi parmi les alcools aromatiques tels que l'alcool benzylique ; les alcools gras liquides , notamment en C₁₀-C₃₀ ; les polyols modifiés ou non tels que le glycérol, le glycol, le propylène glycol, le dipropylène glycol, le butylène glycol, le butyle diglycol ; les silicones volatiles telles que la cylopentasiloxane, la cyclohexasiloxane, les polydiméthylsiloxanes modifiées ou non par des fonctions alkyle et/ou amine et/ou imine et/ou fluoroalkyl et/ou carboxylique et/ou bétaïne et/ou ammonium quaternaire, les polydiméthylsiloxanes modifiées liquides ; les huiles minérales, organiques ou végétales ; les alcanes et plus particulièrement les alcanes de C₅ à C₁₀ ; les acides gras liquides ; les esters gras liquides et plus particulièrement les benzoates ou les salicylates d'alcool gras liquides.

Le solvant organique est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ; ou encore des composés organiques tels que des alcanes en C₅-C₁₀, l'acétone, la méthyléthylcétone, les esters d'acides en C₁-C₂₀ liquides et d'alcools en C₁-C₈ tels que l'acétate de méthyle, l'acétate de butyle, l'acétate d'éthyle et le myristate d'isopropyle, le diméthoxyéthane, le diéthoxyéthane, les alcools gras liquides en C₁₀-C₃₀ tels que l'alcool oléique, les esters d'alcools gras en C₁₀-C₃₀ liquides tels que les benzoates d'alcool gras en C₁₀-C₃₀ et leurs mélanges ; l'huile de polybutène, l'isononanoate d'isononyle, le malate d'isostéaryle, le tétra-isostéarate de pentaérythrityle, le trimélate de tridécyle, le mélange cyclopentasiloxane (14,7 % en poids)/polydiméthylsiloxane dihydroxylé en positions α et γ (85,3 % en poids) ; et leurs mélanges.

Selon un mode de réalisation préféré, le solvant organique est constitué par une silicone ou un mélange de silicone tels que les polydiméthylsiloxanes liquides et les polydiméthylsiloxanes modifiées liquides, la viscosité de la silicone et/ou du mélange de silicone à 25 °C étant comprise entre 0,1 est et 1 000 000 cst, et plus préférentiellement entre 1 est et 30 000 est.

On citera de préférence les huiles et mélanges d'huiles suivantes :
- le mélange de polydiméthylsiloxane alpha-omega-dihydroxylé/cyclopentadiméthylsiloxane (14,7/85,3) commercialisé par Dow Corning sous le nom de DC 1501 Fluid,
- le mélange de polydiméthylsiloxane alpha-omega-dihydroxylé/polydiméthylsiloxane commercialisé par Dow Corning sous le nom de DC 1503 Fluid,
- le mélange de diméthicone/cyclopentadiméthylsiloxane commercialisé par Dow Corning sous le nom de DC 1411 Fluid ou celui commercialisé par Bayer sous le nom SF1214 ;
- la cyclopentadiméthylsiloxane commercialisée par Dow Corning sous le nom de DC 245 Fluid ;
et les mélanges respectifs de ces huiles.

La composition de l'invention peut contenir, outre le ou les solvants organiques liquides, de l'eau. Cependant, selon un mode de réalisation particulier, la composition de l'invention est anhydre c'est-à-dire contenant moins de 1 % en poids d'eau par rapport au poids total de la composition.

Le ou les solvants organiques et l'eau, lorsqu'elle est présente, représentent généralement de 0,01 à 99 %, de préférence de 50 à 99 % en poids par rapport au poids total de la composition.

Le milieu de la composition de l'invention peut aussi se présenter sous forme d'une émulsion et/ou être encapsulé, le(s) monomère(s) cyanoacrylate(s) étant maintenu(s) dans un milieu anhydre jusqu'au moment de l'utilisation. Lorsque le milieu est une émulsion, cette émulsion est par exemple constituée par une phase dispersée ou continue qui peut être constituée par de l'eau, des alcools aliphatiques en C₁-C₄ ou leurs mélanges et une phase organique anhydre comprenant le(s) monomère(s). Dans le cas des capsules ou microcapsules, la capsule peut contenir le(s) monomère(s) dans un milieu anhydre et être dispersée dans un milieu anhydre tel que défini précédemment, de l'eau, des alcools aliphatiques en C₁-C₄ ou leurs mélanges.

La composition cosmétique de l'invention peut également contenir des additifs cosmétiques usuels choisis parmi cette liste non exhaustive tels que les agents réducteurs, les agents oxydants, les séquestrants, les agents épaississants polymériques ou non, les agents hydratants, les agents émollients, les bases organiques ou minérales, les plastifiants, les filtres solaires, les azurants optiques, les colorants d'oxydation, les charges minérales, les argiles, les minéraux colloïdaux, les métaux colloïdaux, les particules de semi-conducteurs de type «puits quantiques» à base de métaux ou de silicium, un composé photo ou thermochromique, les agents nacrant, les parfums, les peptisants, les conservateurs, les protéines, les vitamines, les agents antipelliculaires, les polymères anioniques, cationiques ou amphotères fixants ou non, les agents conditionneurs non polymériques tels que les tensioactifs cationiques.

Les formulations peuvent se présenter sous différentes formes galéniques tels qu'une lotion, une mousse aérosol, un après shampooing ou un shampooing, un gel, ou une cire. Les compositions peuvent être contenues dans un flacon pompe, ou un spray aérosol. Les compositions de l'invention après application sur la chevelure peuvent être rincées ou non.

Lorsque la composition est contenue dans un aérosol, elle peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non (butane, propane, isobutane) et leurs mélanges.

Selon le procédé de l'invention, la composition de l'invention est appliquée sur les fibres kératiniques, en particulier les fibres kératiniques humaines telles que les cheveux, en présence d'un agent nucléophile.

Selon un mode de réalisation particulier du procédé de l'invention, l'agent nucléophile susceptible d'initier la polymérisation du monomère cyanoacrylate peut être appliqué au préalable sur les fibres kératiniques. L'agent nucléophile peut être utilisé pur, en solution, sous forme d'une émulsion ou être encapsulé. Il peut aussi être ajouté à la composition anhydre au moment de l'emploi juste avant l'application sur les fibres kératiniques.

De préférence, cet agent nucléophile est l'eau. Cette eau peut être apportée par exemple par humidification préalable des fibres kératiniques. Elle peut aussi être ajoutée directement dans la composition avant application.

Selon un mode de réalisation particulier, il est possible de moduler la cinétique de polymérisation en humidifiant préalablement la fibre à l'aide d'une solution aqueuse dont le pH a été ajusté à l'aide d'une base, d'un acide ou d'un mélange acide/base. L'acide et/ou la base peuvent être inorganique ou organique.

Selon un mode de réalisation particulier, lorsque le polyuréthane non-ionique se présente sous forme de solution ou dispersion aqueuse ou éthanolique, ce polymère peut alors être appliqué sur le cheveu sous la forme d'un prétraitement.

Selon un autre mode de réalisation, le procédé de traitement des fibres kératiniques de l'invention peut s'appliquer à la coloration capillaire, lorsque la composition comprend des pigments. Un mode de réalisation préféré consiste à appliquer soit la composition selon l'invention contenant lesdits pigments à partir d'une même composition, soit le procédé de coloration capillaire peut être mis en oeuvre en plusieurs étapes : une première étape qui consiste à appliquer une composition contenant le ou les pigments sur les fibres et une seconde étape qui consiste à appliquer une composition selon l'invention contenant entre autre le monomère cyanoacrylate, l'agent nucléophile étant présent dans la composition contenant le pigment ou dans une composition séparée.

Selon cette variante, la composition cosmétique contenant le ou les pigments est de préférence une solution aqueuse de pigments ce qui permet une humidification de la fibre et l'initiation de la polymérisation lorsque la composition selon l'invention est appliquée.

Le procédé de l'invention peut comprendre des étapes additionnelles intermédiaires ou finales telles que l'application d'un produit cosmétique, une étape de rinçage, une étape de séchage. Le séchage peut être effectué au casque, au sèche cheveux et/ou au fer à lisser. En particulier, l'application des compositions conformes à l'invention peut être suivie d'un rinçage.

Il est aussi possible de réaliser des applications multiples de la composition de l'invention afin d'obtenir une superposition de couches pour atteindre des propriétés spécifiques du dépôt en termes de nature chimique, résistance mécanique, épaisseur, aspect, toucher.

Afin d'améliorer entre autre l'adhésion du poly(cyanoacrylate) selon l'invention formé in situ, la fibre peut être prétraitée avec tous types de polymères.

Pour moduler la cinétique de polymérisation anionique, on peut également augmenter la nucléophilie de la fibre par transformation chimique des fibres kératiniques. A titre d'exemple, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants: thiosulfate de sodium anhydre, métabisulfite de sodium en poudre, thiourée, sulfite d'ammonium, acide thioglycolique, acide thiolactique, thiolactate d'ammonium, mono-thioglycolate de glycérol, thioglycolate d'ammonium, thioglycérol, acide 2,5-dihydroxybenzoique, di-thioglycolate de diammonium, thioglycolate de strontium, thioglycolate de calcium, formo-sulfoxylate de zinc, thioglycolate d'isooctyle, dl-cystéine, thioglycolate de monoéthanolamine.

La présente invention concerne également l'utilisation d'une composition cosmétique telle que décrite précédemment pour le traitement des cheveux, et notamment pour leur conditionnement.

Plus particulièrement, lorsque la composition cosmétique comprend au moins un pigment, la composition cosmétique peut être utilisée pour le conditionnement et la coloration des cheveux.

L'invention a encore pour objet un dispositif à plusieurs compartiments ou kit, comprenant un premier compartiment, comprenant une composition selon l'invention, et un second compartiment, comprenant un agent nucléophile.

L'invention a pour autre objet un dispositif à plusieurs compartiments ou kit, comprenant un premier compartiment, comprenant le polyuréthane non-ionique tel que défini ci-dessus, un second compartiment comprenant au moins un monomère cyanoacrylate tel que décrit ci-dessus; l'agent nucléophile étant éventuellement présent dans l'un des deux compartiments pré-cités ou dans un troisième compartiment, chaque compartiment pouvant éventuellement contenir un solvant organique liquide.

Les exemples qui suivent sont destinés à illustrer l'invention, sans présenter un caractère limitatif.

### Exemple 1 :

La composition A suivante est réalisée :

| | |
|---|---|
| ACULYN 44* de ROHM ET HAAS | 10 g |
| Eau | 90 g |

| | |
|---|---|
| *solution de polyuréthane à 35 % dans un mélange d'eau et de propylène glycol | |

La composition suivante B est réalisée :

| | |
|---|---|
| DC 1501 Fluid | 20 g |
| DC 245 Fluid | 44,75 g |
| Méthylheptylcyanoacrylate | |
| commercialisé par Chemence | 10 g |
| Acide acétique | 0,25 g |

0,5 g de la composition A est appliqué sur une mèche de 1 g de cheveux propre et humide. La mèche peut être éventuellement séchée. 0,5 g de la composition B est appliqué ensuite. Après 15 minutes de pause, la mèche est séchée au sèche-cheveux pendant 2 minutes. Les gainages obtenus sont rémanents et épais, ce qui donne une sensation d'enrobage ou de masse qui persiste après shampooing.

### Exemple 2 : gainage coloré

La composition A suivante est réalisée :

| | |
|---|---|
| ACULYN 44* de ROHM ET HAAS | 10 g |
| Eau | 90 g |

| | |
|---|---|
| *solution de polyuréthane à 35 % dans un mélange d'eau et de propylène glycol | |

La composition suivante B' est réalisée :

| | |
|---|---|
| DC 1501 Fluid | 20 g |
| DC 245 Fluid | 44,75 g |
| Nacre mica enrobé d'oxyde de fer brun | |
| commercialisée par Eckart sous le Prestige Bronze | 10 g |
| Méthylheptylcyanoacrylate | |
| commercialisé par Chemence | 10 g |
| Acide acétique | 0,25 g |

0,5 g de la composition A est appliqué sur une mèche de 1 g de cheveux propre et humide. La mèche peut être éventuellement séchée. 0,5 g de la composition B' est appliqué ensuite. Après 15 minutes de pause, la mèche est séchée au sèche-cheveux pendant 2 minutes. Les gainages obtenus sont rémanents et épais, ce qui donne une sensation d'enrobage ou de masse qui persiste après shampooing.

## Revendications

1. Produit cosmétique pour le traitement des fibres kératiniques, en particulier des fibres kératiniques humaines telles que les cheveux, comprenant au moins deux composés, **caractérisé en ce qu'**il comprend :
- comme premier composé, un monomère cyanoacrylate polymérisable, et
- comme deuxième composé, un polyuréthane non ionique,
lesdits composés étant présents dans une même composition ou sous forme séparée, et étant destinés à être appliqués au moment de l'emploi, soit conjointement, soit séparément, de façon simultanée ou séquentielle dans le temps.

2. Produit cosmétique selon la revendication 1, **caractérisée par le fait qu'**il comprend dans la même composition :
- au moins un monomère cyanoacrylate polymérisable, et
- au moins un polyuréthane non ionique.

3. Composition cosmétique selon la revendication 2, **caractérisée en ce que** le monomère cyanoacrylate est choisi parmi les monomères de formule (I) : 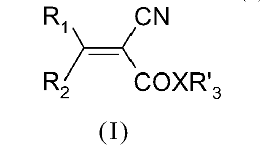 dans laquelle :
X désigne NH, S ou O,
R₁ et R₂ désignent chacun, indépendamment l'un de l'autre, un groupe peu ou non électro-attracteur (peu ou non inductif-attracteur) tel que :
- un atome d'hydrogène,
- un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
- un résidu polyorganosiloxane modifié ou non,
- un groupement polyoxyalkylène,
R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en C₁-C₁₀,
R'₃ représente un atome d'hydrogène ou un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en C₁-C₁₀.

4. Composition cosmétique selon la revendication 3, **caractérisée en ce que** le monomère cyanoacrylate est choisi parmi les monomères de formule (IV) : 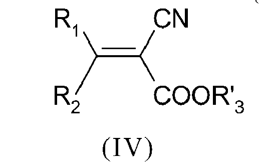 dans laquelle R'₃ représente un radical alkyle en C₁-C₁₀ ou alcoxy(C₁-C₄) alkyle(C₁-C₁₀).

5. Composition cosmétique selon la revendication 4, **caractérisée en ce que** le monomère cyanoacrylate est choisi parmi le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le cyanoacrylate d'allyle, le cyanoacrylate de 2-méthoxypropyle, le 2-cyanoacrylate de 2-méthoxyéthyle, le 2-cyanoacrylate de 2-méthylheptyle, le 2-cyanoacrylate de 2-propoxyéthyle, le 2-cyanoacrylate de n-octyle et le cyanoacrylate d'iso-amyle.

6. Composition cosmétique selon la revendication 5, **caractérisée en ce que** le monomère cyanoacrylate est choisi parmi les cyanoacrylates d'alkyle en C₆-C₁₀.

7. Composition cosmétique selon la revendication 6, **caractérisée en ce que** le monomère cyanoacrylate est choisi parmi les monomères les cyanoacrylates d'octyle de formule (V) et leurs mélanges : 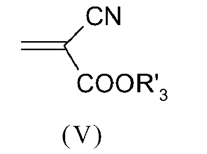
dans laquelle : R'₃ = -(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

8. Composition cosmétique selon l'une quelconque des revendications précédentes, **caractérisé en ce que** le ou les monomères cyanoacrylates sont fixés de façon covalente sur des supports tels que des polymères, des oligomères ou des dendrimères.

9. Composition cosmétique selon l'une quelconque des revendications précédentes, **caractérisée en ce que** la teneur en monomère cyanoacrylate est comprise entre 0,1 et 99,9 % en poids par rapport au poids total de la composition.

10. Composition cosmétique selon l'une quelconque des revendications précédentes, **caractérisé en ce que** le polyuréthane non-ionique est associatif.

11. Composition cosmétique selon l'une quelconque des revendications 1 à 9, **caractérisé en ce que** le polyuréthane non-ionique est non associatif.

12. Composition de traitement cosmétique des matières kératiniques selon la revendication 11, **caractérisée en ce que** le polyuréthane non associatif comprend un motif répétitif de base répondant à la formule générale (VI) :
- O - B - O - CO - NH - R - NH - CO - (VI)
dans laquelle :
- B est un groupe hydrocarboné divalent en C₁ à C₃₀, ce groupe étant substitué ou non par un groupement comportant une ou plusieurs fonctions acides carboxyliques et/ou une ou plusieurs fonctions acides sulfoniques, lesdites fonctions acides carboxyliques et/ou sulfoniques étant sous forme libre ou bien neutralisées partiellement ou totalement par une base minérale ou organique, et
- R est un groupe divalent choisi parmi les groupes hydrocarbonés aliphatiques en C₁ à C₂₀, cycloaliphatiques en C₃ à C₂₀ et aromatiques en C₆ à C₂₀, ou leurs combinaisons, ces groupes étant substitués ou non.

13. Composition cosmétique selon la revendication 12, **caractérisée en ce que** le polyuréthane non associatif est un copolymère acide diméthylolpropionique/isophorone-diisocyanate/néopentylglycol/polyesterdiols.

14. Composition cosmétique selon l'une quelconque des revendications 11 à 13, **caractérisée en ce que** le polyuréthane non associatif comprend en outre un motif répétitif de base répondant à la formule générale (VII) :
- O - P - O - CO - NH - R - NH - CO - (VII)
dans laquelle :
- P est un segment polysiloxanique, et
- R est un groupe divalent choisi parmi les groupes hydrocarbonés aliphatiques en C₁ à C₂₀, cycloaliphatiques en C₃ à C₂₀ et aromatiques en C₆ à C₂₀, ou leurs combinaisons, ces groupes étant substitués ou non.

15. Composition cosmétique selon la revendication 14, **caractérisée en ce que** le polyuréthane non associatif est un copolymère acide diméthylolpropionique/isophorone-diisocyanate/néopentylglycol/polyesterdiols/diamine siliconée.

16. Composition cosmétique selon la revendication 10, **caractérisée en ce que** le polyuréthane associatif est un copolymère acrylique comprenant :
a) environ 40 à 99,5 % en poids d'un monomère à insaturation monoéthylénique non tensioactif et
b) environ 0,5 à 60 % en poids d'un monomère uréthane non-ionique qui est le produit de réaction d'un tensioactif non-ionique monohydrique avec un monoisocyanate à insaturation monoéthylénique.

17. Composition cosmétique selon la revendication 10, **caractérisée en ce que** le polyuréthane associatif non ionique est un polyuréthane-polyéther susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

18. Composition cosmétique selon la revendication 17, **caractérisée en ce que** le polyuréthane associatif non ionique est un polycondensat de polyéthylèneglycol à 150 ou 180 moles d'oxyde d'éthylène, d'alcool stéarylique et de méthylène-bis(4-cyclohexylisocyanate) ou un polycondensat de polyéthylèneglycol à 150 ou 180 moles d'oxyde d'éthylène, d'alcool décylique et de méthylène-bis(4-cyclohexylisocyanate).

19. Composition cosmétique selon l'une quelconque des revendications précédentes, **caractérisée en ce que** la teneur en polyuréthane non-ionique est comprise entre 0,1 et 99,9 % en poids par rapport au poids total de la composition.

20. Composition cosmétique selon l'une quelconque des revendications précédentes, **caractérisée par le fait qu'**elle comprend en outre au moins un pigment.

21. Composition cosmétique selon la revendication 20, **caractérisée par le fait que** le ou les pigments est ou sont sous forme de poudre ou de pâte pigmentaire.

22. Composition cosmétique selon la revendication 20 ou 21, **caractérisée par le fait que** le pigment est un pigment minéral choisi parmi le dioxyde de titane, traité ou en surface, les oxydes de zirconium ou de cérium, les oxydes de fer ou de chrome, le violet de manganèse, le bleu outremer, l'hydrate de chrome et le bleu ferrique.

23. Composition cosmétique selon la revendication 20 ou 21, **caractérisée par le fait que** le pigment est un pigment organique choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

24. Composition cosmétique selon la revendication 20, **caractérisée par le fait que** le pigment est un pigment composite composé de particules comportant un noyau inorganique, au moins un liant assurant la fixation des pigments organiques sur le noyau, et au moins un pigment organique recouvrant au moins partiellement le noyau.

25. Composition cosmétique selon la revendication 20, **caractérisée par le fait que** le pigment est une laque constituée par un substrat inorganique choisi par l'alumine, la silice, le borosilicate de calcium et de sodium ou le borosilicate de calcium et d'aluminium, et l'aluminium, et l'aluminium sur lequel est adsorbé un colorant.

26. Composition cosmétique selon la revendication 20, **caractérisée par le fait que** le pigment est un pigment à effets spéciaux choisi parmi les pigments nacrés, les pigments à effets interférentiels non fixés sur un substrat, les pigments photochromiques, les pigments thermochromiques et les quantum dots.

27. Composition cosmétique selon la revendication 26, **caractérisée par le fait que** le ou les pigments nacrés sont choisis parmi le mica recouvert de titane ou d'oxychlorure de bismuth, le mica recouvert de titane ou d'oxydes de fer, le mica recouvert d'oxyde de fer, le mica recouvert de titane et de bleu ferrique ou d'oxyde de chrome, le mica recouvert de titane et d'un pigment organique tel que défini à la revendication 23, les pigments nacrés à base d'oxychlorure de bismuth.

28. Composition cosmétique selon la revendication 27, **caractérisée par le fait que** le pigment nacré est le mica recouvert d'oxyde de fer.

29. Composition cosmétique selon l'une quelconque des revendications 20 à 28, **caractérisée par le fait que** le ou les pigments est ou sont présents en une quantité allant de 0,05 à 50 % en poids, de préférence en une quantité allant de 0,1 à 35 % en poids.

30. Composition cosmétique selon l'une quelconque des revendications précédentes, **caractérisée en ce qu'**elle comprend en outre au moins un inhibiteur de polymérisation.

31. Composition cosmétique selon la revendication 30, **caractérisée par le fait que** la composition comprend au moins un acide minéral ou organique choisi parmi l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique, l'acide trifluoroacétique, l'acide octanoïque, l'acide heptanoïque et l'acide hexanoïque.

32. Composition cosmétique selon la revendication 31, **caractérisée par le fait que** l'acide est l'acide acétique.

33. Composition cosmétique selon l'une quelconque des revendications 30 à 32, **caractérisée par le fait que** le ou les inhibiteurs de polymérisation sont présent en une quantité allant de 10 ppm à 30 % en poids, de préférence en une quantité allant de 10 ppm à 15 % en poids.

34. Composition cosmétique selon l'une quelconque des revendications précédentes, **caractérisée par le fait qu'**elle est anhydre.

35. Composition cosmétique selon la revendication 34, **caractérisée par le fait qu'**elle comprend au moins un solvant organique liquide choisi parmi les alcools aromatiques, les alcools gras, les polyols modifiés ou non, les silicones volatiles ou non, les huiles minérales, organiques ou végétales, les cires oxyéthylénées ou non, les paraffines, les alcanes, les acides gras, les amides grasses, les esters gras.

36. Composition cosmétique selon la revendication 35, **caractérisée par le fait que** le solvant organique est constitué de cyclopentadiméthyl siloxane.

37. Composition selon l'une quelconque des revendications précédentes, **caractérisée par le fait qu'**elle contient des additifs cosmétiques usuels choisis parmi les agents réducteurs, les agents oxydants, les séquestrants, les agents épaississants polymériques ou non, les agents hydratants, les agents émollients, les bases organiques ou minérales, les plastifiants, les filtres solaires, les azurants optiques, les colorants d'oxydation, les charges minérales, les argiles, les minéraux colloïdaux, les métaux colloïdaux, les nano-particules de semi-conducteurs de type «puits quantiques» à base de métaux ou de silicium, un composé photo ou thermochromique, les parfums, les peptisants, les conservateurs, les protéines, les vitamines, les agents antipelliculaires, les polymères anioniques, cationiques ou amphotères fixants ou non, les agents conditionneurs non polymériques tels que les tensioactifs cationiques.

38. Composition cosmétique selon l'une quelconque des revendications précédentes, **caractérisée par le fait qu'**elle comprend en outre au moins un agent nucléophile.

39. Composition cosmétique selon la revendication 38, **caractérisée par le fait que** l'agent nucléophile est de l'eau.

40. Procédé de traitement cosmétique des fibres kératiniques teintes artificiellement, notamment les fibres kératiniques humaines et plus particulièrement les cheveux humains, **caractérisé par le fait que** l'on applique sur lesdites fibres au moins une composition cosmétique telle que définie dans la revendications 1 à 37 en présence d'au moins un agent nucléophile tel que défini à la revendication 38 ou 39 ; ledit agent nucléophile étant mélangé au moment de l'emploi dans ladite composition ou appliqué séparément.

41. Procédé de traitement selon la revendication 40, **caractérisé par le fait que** l'on applique sur lesdites fibres une composition comprenant le polyuréthane non-ionique tel que défini à l'une quelconque des revendications 10 à 19, puis l'on applique sur lesdites fibres une composition comprenant le monomère cyanoacrylate tel que défini à l'une quelconque des revendications 2 à 8, l'agent nucléophile se trouvant dans l'une ou l'autre des deux compositions.

42. Procédé de coloration capillaire, **caractérisé par le fait qu'**il comprend une première étape consistant à appliquer une composition contenant au moins un pigment tel que défini à l'une quelconque des revendications 20 à 29 sur lesdites fibres kératiniques et une seconde étape consistant à appliquer une composition cosmétique telle que définie à l'une quelconque des revendications 1 à 19 et 30 à 37 , l'agent nucléophile étant présent dans la composition comprenant le pigment ou dans une composition séparée.

43. Procédé de coloration selon la revendication 42, **caractérisé par le fait que** la composition comprenant le ou les pigments est une composition aqueuse de pigments et la composition telle que définie à l'une quelconque des revendications 1 à 19 et 30 à 37 est anhydre.

44. Utilisation d'une composition cosmétique selon l'une quelconque des revendications 1 à 39 pour le traitement des cheveux, de préférence pour le conditionnement des cheveux.

45. Utilisation d'une composition cosmétique selon l'une quelconque des revendications 20 à 39 pour la coloration des cheveux.

46. Dispositif à plusieurs compartiments ou kit, comprenant un premier compartiment, comprenant une composition telle que définie à l'une quelconque des revendications 1 à 37, et un second compartiment, comprenant au moins un agent nucléophile.

47. Dispositif à plusieurs compartiments ou kit, comprenant un premier compartiment, comprenant le polyuréthane non-ionique tel que défini à l'une quelconque des revendications 10 à 19, un second compartiment comprenant au moins un monomère cyanoacrylate tel que décrit à l'une quelconque des revendications 3 à 9 ; l'agent nucléophile étant présent dans l'un des deux compartiments pré-cités ou dans un troisième compartiment, chaque compartiment pouvant éventuellement contenir un solvant organique liquide.
